# EUROPEAN PATENT SPECIFICATION

(11) **EP 3 858 308 B1**
(45) Date of publication and mention of the grant of the patent: **30.08.2023**
(21) Application number: 19864268.8
(22) Date of filing: 10.07.2019
(51) Int. Cl.: A61F 13/15, A61F 13/47, A61F 13/475, A61F 13/56

(54) **INDIVIDUALLY PACKAGED ABSORBENT ARTICLE**
EINZELN VERPACKTER SAUGFÄHIGER ARTIKEL
ARTICLE ABSORBANT EMBALLÉ INDIVIDUELLEMENT

(30) Priority: 27.09.2018 JP 2018182208
(43) Date of publication of application: 04.08.2021
(73) Proprietor: DAIO PAPER CORPORATION, Shikokuchuo-shi Ehime 799-0492 (JP)
(72) Inventor: KUNIMORI, Ayano, Sakura-shi, Tochigi 329-1411 (JP)
(74) Representative: Epping - Hermann - Fischer
(86) International application number: PCT/JP2019/027343
(87) International publication number: WO 2020/066221

(56) References cited:
- WO-A1-2017/221941
- JP-A- 2001 037 804
- JP-A- 2002 362 624
- JP-A- 2008 237 384
- JP-A- 2009 136 341
- JP-A- 2010 178 932
- JP-A- 2012 050 498
- JP-A- 2017 000 329
- JP-A- 2017 042 471
- JP-A- 2018 083 015

## Description

### TECHNICAL FIELD

The present invention relates to an individually wrapped absorbent article.

### BACKGROUND OF THE INVENTION

As absorbent articles such as sanitary napkins, panty liners, incontinence pads, and the like, it is known that an absorbent is sandwiched between a liquid-permeable top sheet and a liquid-impermeable back sheet. Some of these absorbent articles are adapted for prolonged use or for short periods of time when large volumes of body fluid are discharged, with a main body of an absorbent article extending in a rear area and with flaps projecting from both sides of the rear side thereby extending the rear area.

Absorbent articles are typically provided as an individual wrapped article wrapped with a wrapping sheet. Here, in the case of aforementioned absorbent article having the extended rear area, the flaps protruding from both sides are folded toward the main body of the absorbent article, and then tri-folded or quad-folded in the lengthwise direction and wrapped (for example, FIG. 4 in Patent Document 1).

### RELATED-ART DOCUMENT

### Patent Document

Patent Document 1: Japanese Patent Application Laid-Open No. 2003-339764

Further, JP 2018 083015 A and JP 2009 136341 A refer to absorbent articles including a main body having a liquid-permeable top sheet, a liquid-impervious back sheet, and an absorbent body provided between the top sheet and the back sheet.

### SUMMARY OF THE INVENTION

### Problems to be solved by the Invention

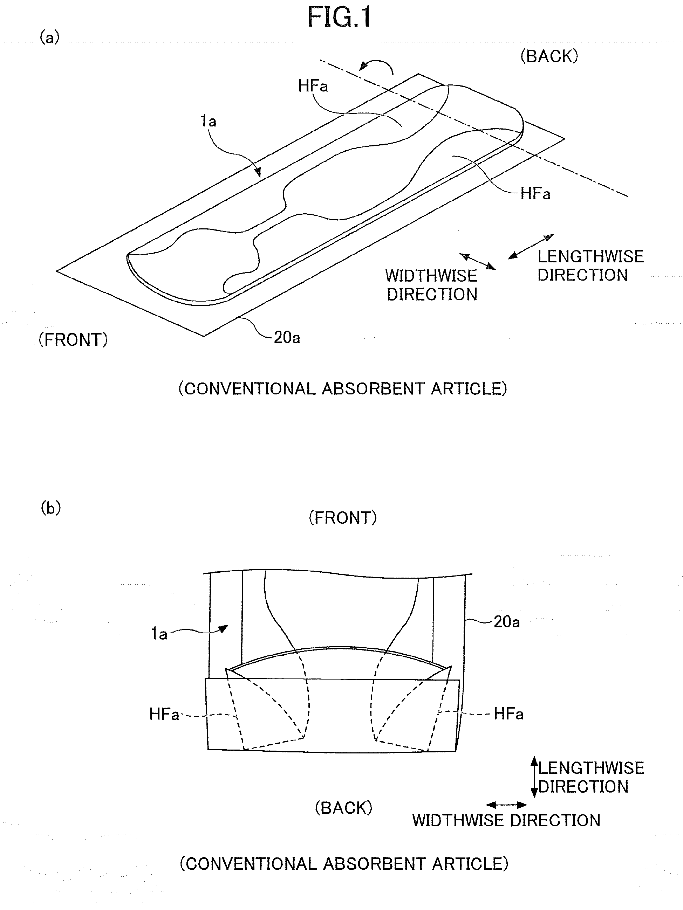

However, in the configuration described in Patent Document 1, when the rear area is folded, the flaps that have been folded in the widthwise direction may inadvertently spread outward in the widthwise direction (Fig. 1(a) and (b)). In such cases, the flaps might extend out of the wrapping sheet or overlap with the sealing position of the wrapping sheet. Therefore, the size of the individually wrapped may become larger than intended when the width of the wrapping sheet is increased to address such issues. Therefore, there is a need for a configuration in which the flaps can be folded so as not to spread outward in the widthwise direction in the absorbent article with the expanded rear area.

In view of the foregoing, one aspect of the invention is to provide an absorbent article that is compactly wrapped by reducing a spread of flaps in a widthwise direction upon folding.

### Means for Solving the Problem

In the invention, an individually wrapped absorbent article includes an absorbent article being a sanitary napkin and having a main body, the main body including a liquid-impermeable back sheet, an absorbent, and a liquid-permeable top sheet that are laminated in aforementioned order, the main body is the absorbent article having a predetermined length in a lengthwise direction and a predetermined width in a direction perpendicular to the lengthwise direction; and a wrapping sheet that wraps the absorbent article individually, wherein the absorbent article includes a middle area that includes a portion corresponding to a body fluid excretion orifice of a wearer upon wearing the absorbent article, and a rear area that is adjacently behind the middle area and extends to a rear end of the absorbent article, wherein the absorbent article includes hip-hold flaps projecting laterally from the main body in the rear area, and an outline of the hip-hold flaps has convex portions with a radius of curvature of 5 to 500 mm, wherein anti-slipping portions are disposed on a back surface of the hip-hold flaps, and wherein the hip-hold flaps are folded with a top sheet side inward along lengthwise direction folding lines extending substantially in the lengthwise direction, and the rear area is then folded with the top sheet side inward along a widthwise direction folding line extending substantially in the widthwise direction through the anti-slipping portions and apexes of the convex portions. A flexible portion is formed in the main body, and the widthwise direction folding line passing through the flexible portion. The flexible portion includes a portion having a reduced thickness of the absorbent.

### Effects of the Invention

The invention is capable of providing an absorbent article that is compactly wrapped by reducing a spread of flaps in a widthwise direction upon folding.

### BRIEF DESCRIPTION OF THE DRAWINGS

FIG. 1 is a view illustrating a part of a process in which a conventional absorbent article is folded;
FIG. 2A is a partially cutaway plan view of an absorbent article in one embodiment of the invention;
FIG. 2B illustrates a cross-sectional view of line I-I of FIG. 2A;
FIG. 3A illustrates a process of forming an individually wrapped absorbent article according to one aspect of the invention;
FIG. 3B illustrates a process of forming an individually wrapped absorbent article according to one aspect of the invention;
FIG. 3C illustrates a process of forming an individually wrapped absorbent article according to one aspect of the invention;
FIG. 3D illustrates a process of forming an individually wrapped absorbent article according to one aspect of the invention;
FIG. 3E illustrates a process of forming an individually wrapped absorbent article according to one aspect of the invention;
FIG. 4 illustrates a variation of an absorbent article in one embodiment of the invention; and
FIG. 5 illustrates a variation of an absorbent article in one embodiment of the invention.

### DETAILED DESCRIPTION OF THE INVENTION

Hereinafter, embodiments of the present invention will be described in detail with reference to the drawings. In each drawing, unless otherwise noted, the description of the same or corresponding structure may be omitted with the same reference numerals. Also, the drawings are schematic to aid in understanding the invention.

### (Basic Structure of Absorbent Articles)

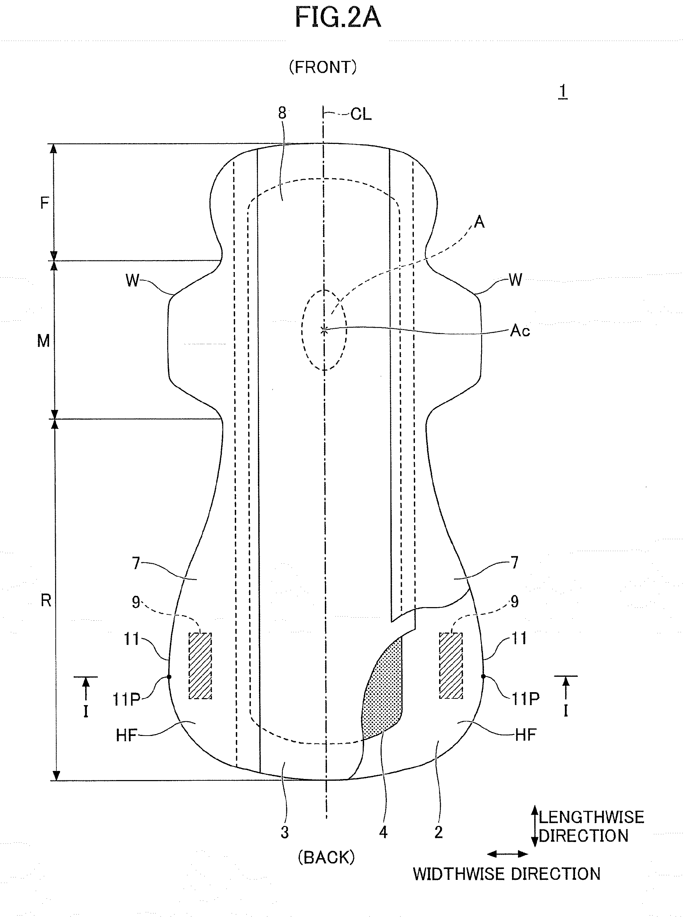

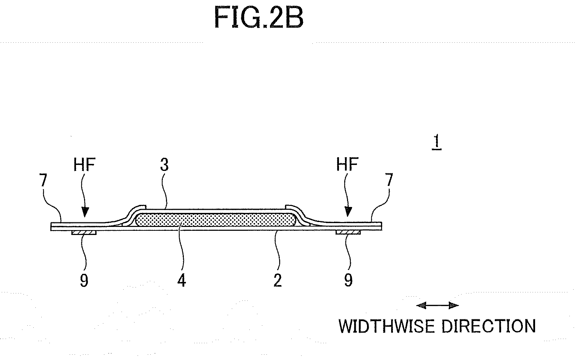

FIG. 2A illustrates a partially cutaway plan view of absorbent article 1 in one embodiment of the invention of the present invention. FIG. 2B illustrates a cross-sectional view of line I-I of absorbent article 1. As illustrated in FIGS. 2A and 2B, the absorbent article 1 includes a main body (absorbent article body) 8 having a liquid-impermeable back sheet 2, a liquid-permeable top sheet 3, and an absorbent 4 provided between the top sheet 3 and the back sheet 2. When the absorbent article 1 is worn, the top sheet 3 contacts skin (skin side or top side), and the back sheet 2 is fixed to an underwear (underwear side or back side). Both sides of the top sheet 3 of the absorbent article 1 may be provided with side non-woven fabrics 7 and 7 extending in a lengthwise direction so as to overlap with both edges of the widthwise direction of the absorbent 4 in a plan view.

As illustrated in FIG. 2A, a main body 8 has a predetermined length in a lengthwise direction, a predetermined width in a widthwise direction perpendicular to the lengthwise direction, and an overall elongated shape in the lengthwise direction. The width of the main body 8 is substantially constant in the illustrated example but may vary across the lengthwise direction. As illustrated in FIG. 2A, the absorbent article 1 can have a generally symmetrical shape with respect to the centerline (centerline in lengthwise direction) CL extending in a lengthwise direction. The configuration of the absorbent article 1 (including the thickness and density of the absorbent 4) may be substantially symmetrical with the centerline as the axis of symmetry or may be asymmetric.

The length in the lengthwise direction (overall length of the main body 8) of absorbent article 1 is not particularly limited but preferably 250 to 450 mm, and more preferably from 270 to 380 mm. The length of the widthwise direction of the main body 8 (the width of the absorbent 4) is not particularly limited but is preferably 50 to 200 mm, and is more preferably 70 to 120 mm.

The absorbent article 1 has a portion A corresponding to a body fluid excretion orifice corresponding to a body fluid excretion orifice such as a vaginal opening of a wearer when the absorbent article is worn. The portion A corresponding to a body fluid excretion orifice has a center Ac. The center Ac is positioned on the centerline CL in the lengthwise direction and is positioned on the center of the lengthwise direction of the portion A corresponding to a body fluid excretion orifice. The position of the center Ac is 70 to 130 mm away from the front end of the absorbent article 1. Further, the portion A corresponding to a body fluid excretion orifice may have a length (length in the lengthwise direction) of 10 to 50 mm and preferably 20 to 40 mm. The portion A corresponding to a body fluid excretion orifice may have a width (length in the widthwise direction) of 5 to 40 mm and preferably 10 mm to 30 mm. It should be noted that the shape and size of the portion A corresponding to a body fluid excretion orifice illustrated in the drawings are merely examples for ease of explanation.

The absorbent article 1 has a middle area M including a portion corresponding to a body fluid excretion orifice A described above, a front area F in front of the middle area M, and a rear area R in behind of the middle area M. The middle area M corresponds approximately to the wearer's crotch when worn, and the rear area R corresponds approximately to the wearer's buttocks when worn.

The middle area M may have wing-like flaps W and W extending from both sides thereof to securely fix the absorbent article 1 to an underwear upon mounting. The front area F is the area from the front origins or the near the front origins of the wing-like flaps W and W to the front end of the absorbent article. The rear area R is the area from the rear origins or near the rear origins of the wing-like flaps W and W to the rear end of the absorbent article. When the wing-like flaps W and W are provided, the position of the center Ac of the portion A corresponding to a body fluid excretion orifice in the lengthwise direction may be near the center of the sides in the wing-like flaps W and W in the lengthwise direction.

The absorbent article 1 has wing-like flaps W and W. However, the absorbent article 1 need not have wing-like flaps W and W. When it is assumed that the wing-like flaps W and W are formed, the area from the front origins or near the front origins of the wing-like flaps W and W to the front end of the absorbent article is determined as the front area F. When it is assumed that the wing-like flaps W and W are formed, the area from the rear origins or near the rear origins of the wing-like flaps W and W to the rear end of the absorbent article is determined as the rear area R.

The back sheet 2 may be a sheet material having at least a water shielding property, such as an olefin-based resin sheet such as polyethylene or polypropylene. A laminated non-woven fabric made by laminating a non-woven material with a polyethylene sheet or the like or a non-woven sheet made to be substantially liquid-impermeable by interposing a waterproof film with the laminated material or the like can be used. In addition, from the viewpoint of preventing stuffiness, it is further desirable to use a material having moisture permeability. As a waterproof and moisture permeable sheet material, a microporous sheet or the like may be obtained by melt-kneading an inorganic filler into an olefinic-based resin such as polyethylene or polypropylene to form a sheet, followed by stretching the sheet in a monoaxial or a biaxial direction.

The top sheet 3 is a liquid-permeable sheet through which bodily fluids such as menstrual blood, discharge, and urine pass. As the top sheet 3, a porous or non-porous non-woven fabric or a porous plastic sheet is preferably used. As the material fibers constituting the non-woven fabric, for example, olefins such as polyethylene, polypropylene, and the like; synthetic fibers such as polyester, polyamide, and the like; regenerated fibers such as rayon, cupra, and the like; and blended fibers, as well as natural fibers such as cotton and the like can be used alone or in combination. Examples of non-woven fabric processing methods include a span lace method, a spunbond method, a thermal bond method, a meltblown method, a needle punch method, and the like. Among these processing methods, the span lace method is preferably used from the view point of flexibility, the spunbond method is preferably used from the view point of producing a non-woven fabric with high drape properties, and the thermal bond method is preferably used from the view point of producing a non-woven fabric that is bulky and soft. Alternatively, composite fibers such as core-sheath type fibers with high melting point fibers as the core and low melting point fibers as the sheath, side-by-side type fibers, split type fibers, and the like may be used.

The materials of absorbent 4 are not particularly limited as long as the materials are capable of absorbing and retaining body fluid. Cotton pulps and water absorbent polymers are preferably contained in the materials. Examples of the water absorbent polymers include superabsorbent polymer (SAP), superabsorbent polymer fiber (SAF), and a combination thereof. Examples of the pulps include cellulosic fibers such as chemical pulps obtained from woods, dissolved pulps, and the like; and artificial cellulose fibers such as rayon, acetate, and the like. Examples of raw materials for chemical pulps include hardwood, coniferous wood, and the like. Coniferous wood is preferably used because of its long fiber length.

Synthetic fibers may also be mixed with the absorbent 4. Examples of the synthetic fibers include polyolefins such as polyethylene, polypropylene, and the like; polyesters such as polyethylene terephthalate, polybutylene terephthalate, and the like; polyamides such as nylon and the like; and copolymers thereof. Among these, two kinds of the synthetic fibers may be used in combination. Also, composite fibers such as core-sheath type fibers with high melting point fibers as the core and low melting point fibers as the sheath, side-by-side type fibers, split type fibers, and the like may be used. Alternatively, hydrophobic fibers may be surface-treated with a hydrophilic agent to confer affinity to body fluids. The absorbent 4 is preferably manufactured by laminating fibers or an air laid process.

The thickness of the absorbent 4 is in the range of 0.3 to 30 mm and preferably in the range of 1.0 to 15 mm. The absorbent 4 as a whole need not have a uniform thickness across the entire surface, and the area of the portion A corresponding to a body fluid excretion orifice and its proximity may be inflated.

The absorbent 4 may be wrapped with a wrapped sheet formed from crepe paper or non-woven fabric to maintain its shape or the like. The absorbent 4 can be prevented from becoming warped or cracked by surrounding the absorbent with a packaging a packaging sheet. As the packaging sheet, a colorless (that is white) crepe paper or a non-woven material may be used, or a colored packaging sheet may be used. When a colored packaging sheet is used, the color of the packaging sheet can also be reflected as a light color from the top sheet 3. Therefore, the color of the excreted body fluid can be close to the reflected color of the packaging sheet 9 to make it inconspicuous. The packaging sheet can be colored, for example, as a complementary color of body fluids. For example, in an absorbent article (such as a sanitary napkin) configured for the purpose of absorbing blood, the packaging sheet can be colored green, which is the complementary color of red.

The side non-woven fabric 7 may be formed from non-woven fabric materials that are subjected to an appropriate water-repellent treatment or hydrophilic treatment in accordance with the purpose of preventing the body fluid from penetrating or enhancing the feel against the skin. The non-woven materials may be, for example, natural fibers, synthetic fibers, regenerated fibers, and the like. For the water-repellent treatment of the side non-woven fabric 7, a water-repellent agent, such as a silicon-based material or a paraffin-based material, may be used.

At the front end and rear end of the main body 8, the outer edges of the back sheet 2 and the top sheet 3 are bonded by an adhesive method such as hot melt, heat seal, ultrasonic seal, or the like. The side non-woven fabrics 7 and 7 extending from the side of the main body 8 and the back sheet 2 are bonded by at least one of the adhesive methods. Thus, the wing-like flaps W and W described above can be formed in the middle area M, and the hip-hold flaps HF and HF described below can be formed in the rear area R.

The main body 8 may be provided with pressure grooves. The pressure grooves are grooves (also referred to as fitting embosses) that are obtained by laminating the top sheet 3 and the absorbent 4 and then pressurizing both of them together such that the top sheet 3 side is depressed toward the sheet 2. The pressure grooves can be formed by laminating the top sheet 3 onto the absorbent 4 and passing the resulting laminate between a pair of pressurizing rolls. For example, a convex roll on the side of the top sheet 3 of the laminate and a flat roll on the side of the absorbent 4 can be arranged and pressurized by both rolls.

### (Hip-Hold Flaps)

As illustrated in FIG. 2A, the absorbent article 1 in the present embodiment is the rear area R expanded in the lengthwise direction and the widthwise direction. More specifically, the main body 8 extends in the lengthwise direction, and the hip-hold flaps HF and HF extend on both sides of the body 8. The flexibility of the hip-hold flaps HF and HF allows them to be applied along round portion of the buttocks, and also causes them to readily conform to the movements of the buttocks. As illustrated in FIGS. 2A and 2B, in the present embodiment, the hip-hold flaps HF and HF are formed by bonding the side non-woven fabrics 7 and 7 and the back sheet 2, and do not include absorbent. However, the hip-hold flaps HF and HF may contain a thin absorbent as long as the absorbent can have flexibility to align with the round portions of the buttocks.

Thus, the absorbent article 1 in the present embodiment has an extended rear area R. This effectively prevents leakage of body fluid in the rear area R. Accordingly, the absorbent article 1 can be suitably used as a sanitary napkin for long-time or night-time use, because it can accommodate a situation where long hours of use is expected or a situation where a large amount of body fluid is excreted within a short period of time.

As illustrated in FIG. 2A, the outline of the hip-hold flaps HF has convex portions 11 which are curved. That is, each convex portion 11 may have a convex-shaped outline with a center of curvature on the hip-hold flaps HF or the main body 8 in a planar view. The radius of curvature at apexes 11P of the convex portions 11 may be 5 to 500 mm and preferably 35 to 200 mm. As described above, the hip-hold flaps HF have an external shape having a convex portion having a predetermined radius of curvature, so that the flat hip-hold flaps HF readily deform to match the curved surface of buttocks. Accordingly, the hip-hold flaps HF are easily moved from both ends of the convex portions. Therefore, the rear area R can be tightly attached to the curved surface of buttocks to improve the effectiveness of preventing leakage of body fluid in the rear area R. In addition, a feeling of discomfort when the edges of the hip-hold flaps HF and HF contact the skin can be reduced, because the contour is curved.

In the illustrated embodiment, the outline of each hip-hold flap HF is shaped to have one convex portion, but the convex portion of the hip-hold flap HF may be two or more. That is, the outline of the hip-hold flap HF may have irregularities. In such a case, each convex portion preferably has a radius of curvature in the range described above.

The absorbent article 1 may have a maximum width at a position where the hip-hold flap HF has a convex portion 11 (where the convex portion is two or more, the convex portion has the most protruding portion outward in the width direction). This maximum width is preferably 70 to 200 mm and more preferably 100 to 160 mm.

In addition, the position of the convex portion 11 (or the position of the maximum width when there are two or more convex portions) is preferably in a range from the position of the rear area R in the center of the lengthwise direction to the rear direction. This allows the wide part of the hip-hold flaps HF and HF to be positioned near the point where the buttocks groove (gluteal cleft) begins to become shallow and the buttocks groove ends. This prevents leakage of fluid that has moved to the rear through the buttocks groove in the lateral direction. In addition, it is capable of preventing the hip-hold flaps HF and HF from hitting around legs and causing a feeling of discomfort because the wide portion of the hip-hold flaps HF and HF can be moved away from the position where the pressure is easily applied from the legs.

### (Anti-Slipping Portions)

The absorbent article 1 in the present embodiment is provided with anti-slipping portions 9 on the sides of the back sheet 2 for securing the absorbent article 1 to underwear when worn. The anti-slipping portions 9 are preferably disposed on the back sheet 2 side of the main body 8, the wing-shaped flaps W and W, and the hip-hold flaps HF and HF. FIG. 2A illustrates the anti-slipping portions 9 disposed on the back sheet 2 side of the hip-hold flaps HF and the HF. The other anti-slipping portions are omitted.

The materials used for the anti-slipping portions 9 are not particularly limited as long as the absorbent article 1 functions so as not to slip from the underwear. The anti-slipping portions 9 can be formed as an adhesive layer or non-adhesive layer. Examples of adhesive layers include a styrene-based polymer, a tackifier, a plasticizer, and a combination thereof. Examples of the styrene-based polymer include a styrene-ethylene-butylene-styrene block copolymer, a styrene-butylene-styrene block copolymer, a styrene-isobutylene-styrene copolymer, a styrene-butadiene-styrene block copolymer, and the like. One or more types of styrene-butadiene-styrene block copolymers may be used. Among these, a styrene-butadiene-styrene block copolymer is preferably used from the view point of thermostability.

As a tackifier and a plasticizer, a solid material may be used at room temperature. Examples of the tackifiers include C5-based petroleum resins, C9-based petroleum resins, dicyclopentadiene-based petroleum resins, rosin-based petroleum resins, polyterpene resins, terpenephenol resins, and the like. Examples of the plasticizers include monomer plasticizers such as trifrecyl phosphate, dibutyl phthalate, and dioctyl phthalate, as well as polymer plasticizers such as vinyl polymers, polyesters, and the like.

Examples of the materials of the anti-slipping portions 9 that are formed as non-adhesive layers include a material using a base polymer such as natural rubber, styrene-butadiene rubber, silicone rubber, chloroprene rubber, butyl rubber, styrene-isoprene rubber, and the like. The material may be used alone or in combination. Among these, silicone rubber or hydrogenated type elastomers of SIS and SBS are preferably used. A mechanical fastener hook material may be used as the anti-slipping portions 9. The shape of the hook is not particularly limited as long as the hook is securable to the underwear, and a variety of publicly known shapes, such as a sauce shape, mushroom shape, or the like, may be used.

As illustrated in FIG. 2A, the anti-slipping portions 9 may be provided along the lengthwise direction of the hip-hold flaps HF and HF. That is, the anti-slipping portions 9 may be provided along the lengthwise direction of the absorbent article 1. Accordingly, the anti-slipping portions 9 have an elongated shape in the lengthwise direction as illustrated in FIG. 2A.

The length of the anti-slipping portions 9 (length in lengthwise direction) may be from 10 to 100 mm and preferably from 15 to 50 mm. The width (length in widthwise direction) of the anti-slipping portions 9 may be from 0.3 to 20 mm and preferably from 0.5 to 15 mm.

The anti-slipping portions 9 in the hip-hold flaps HF and HF may be provided as a contiguous single area or as a plurality of areas that are discontinuous in the lengthwise direction and/or the widthwise direction.

### (Individually Wrapped Absorbent Articles)

One embodiment of the present invention is an absorbent article (individually wrapped absorbent article) individually wrapped so that it can be easily and sanitarily carried. The individually wrapped absorbent article includes an absorbent article 1 and a wrapping sheet 20 as described above. The individually wrapped absorbent article may be formed by wrapping the absorbent article 1 by folding the wrapping sheet 20, or by folding the wrapping sheet 20 along with the absorbent article 1, after the absorbent article 1 is mounted on the wrapping sheet 20, so that the absorbent article 1 is not exposed.

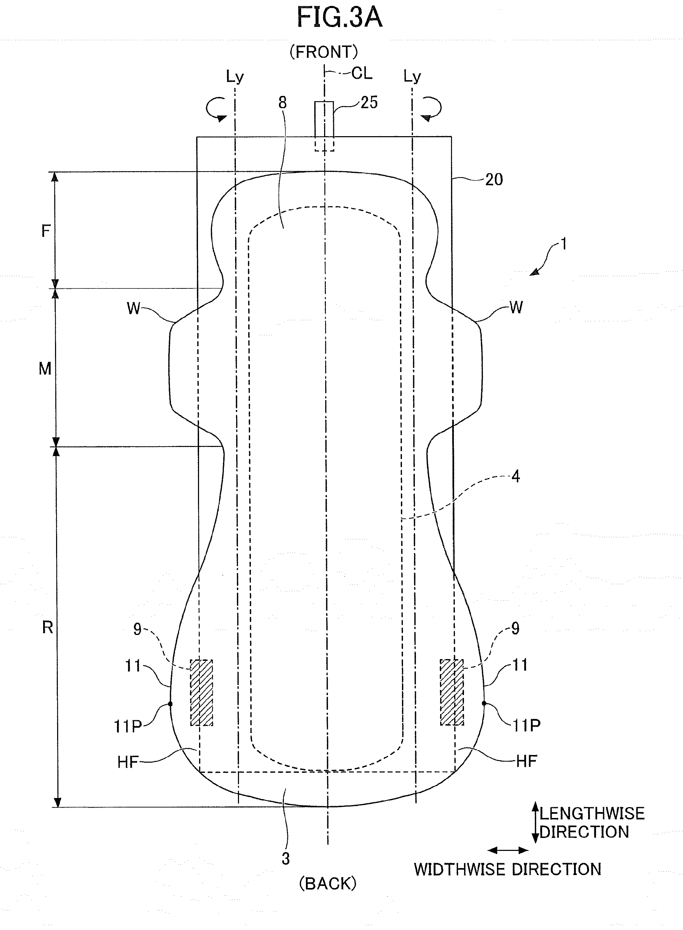

The wrapping sheet 20 is not particularly limited in configuration and size as long as the wrapping sheet can wrap the absorbent article 1. For example, the wrapping sheet 20 may be rectangular in plan view as described below (FIG. 3A).

The wrapping sheet 20 may be a polyolefin-based film such as polypropylene, polyethylene, and the like; a film such as polyester, polyvinyl alcohol, and the like; a non-woven fabric; a laminated non-woven fabric (including a non-woven polylaminated fabric in which a polymeric layer is laminated on one or both surfaces of the non-woven fabric); and the like. The films are preferably used if a design by printing is desirable. The non-woven fabrics are preferably used if a texture, soft feel, or the like are desirable.

### (Folding of Absorbent Articles)

Referring to FIGS. 3A to 3E, the folding and individually wrapping steps of the above-described absorbent article 1 (FIGS. 2A and 2B) with hip-hold flaps HF and HF having an outline with a radius of curvature of 5 to 500 mm will be described. For simplicity of description, the details of the structure of the absorbent article 1 are omitted in FIGS. 3A to 3E.

FIG. 3A illustrates a top view (view from the side of the top sheet 3) of the absorbent article 1 mounted on the wrapping sheet 20 in a direction such that the back sheet 2 faces the wrapping sheet 20. When the absorbent article 1 is folded, first, the hip-hold flaps HF and HF on both sides of the main body 8 are folded with the top sheet 3 inward along the lengthwise direction folding lines Ly and Ly extending in a substantially lengthwise direction.

As used herein, the substantially lengthwise direction may be a direction extending in a lengthwise direction, that is, parallel to the centerline CL of lengthwise direction in the absorbent article 1. Alternatively, a direction may have an angle of 10° or less and preferably 5° or less from the centerline CL of the lengthwise direction. The same shall apply to the substantially widthwise direction. That is, a direction may be parallel to the direction perpendicular to the centerline CL of the lengthwise direction, or a direction may be 10° or less and preferably 5° or less from the direction perpendicular to the centerline CL of the lengthwise direction.

The lengthwise direction folding lines Ly and Ly extend over the hip-hold flaps HF and HF so as to avoid areas where the absorbent 4 is disposed. As illustrated in FIG. 3A, the lengthwise direction folding lines Ly and Ly preferably do not pass over the anti-slipping portions 9 and 9 disposed on the back sheet 2 side of the hip-hold flaps HF and HF. This makes it easier to fold the hip-hold flaps HF and HF in the widthwise direction. In addition, during the subsequent folding in the lengthwise direction, a situation where the absorbent article does not fold readily due to the excessive thickness and excessive bending stiffness can be prevented from occurring. In addition, the lengthwise direction folding lines Ly and Ly may pass over the anti-slipping portions 9 and 9. In this case, the hip-hold flaps HF and HF can be folded stably along the folding position, and thus the shape after the folding is easily retained.

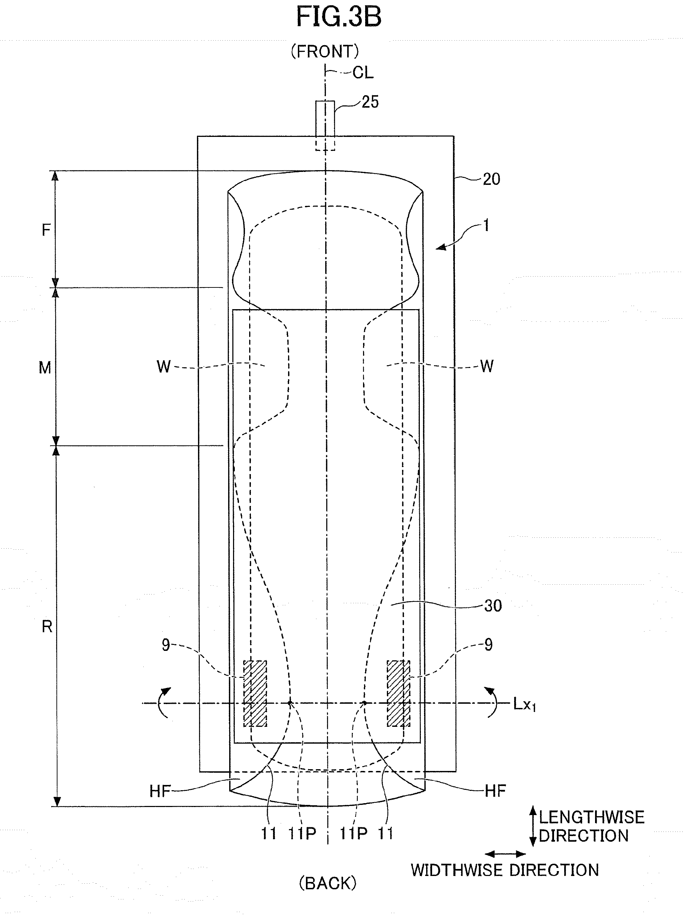

FIG. 3B illustrates a state after the hip-hold flaps HF and HF are folded at the lengthwise direction folding lines Ly and Ly. In this state, the hip-hold flaps HF and HF are positioned inwardly from the ends of the widthwise direction in the wrapping sheet 20.

Beginning from the state illustrated in FIG. 3B, the rear area R of the absorbent article 1 is folded together with the wrapping sheet 20 in the lengthwise direction. First, as illustrated in FIG. 3B, a portion of the absorbent article 1 including the rear end thereof is folded with the top sheet 3 inward along a first-widthwise-direction folding line Lx1 extending in substantially a widthwise direction. At this time, the portion of the absorbent article 1 is folded so that the first-widthwise-direction folding line Lx1 passes through the anti-slipping portions 9 and 9 provided on the hip-hold flaps HF and HF. The portions where the anti-slipping portions 9 are provided are thicker, harder, and more rigid than the portions where the anti-slipping portions are not provided. For this reason, in the portions where the anti-slipping portions 9 are provided and their vicinities, a deformation in association with folding, deflection of the flaps, or slipping from the position folded in the widthwise direction is unlikely to occur.

The hip-hold flaps HF of the present embodiment have an outline having a convex portion having a radius curvature of 5 to 500 mm so that the convex portion can be moved independently upon folding. However, even in this shape, when the first-widthwise-direction folding line Lx1 is folded so as to pass through the anti-slipping portions 9, the hip-hold flaps HF are hardly deflected. Therefore, it is easy to retain the position folding in the widthwise direction. Accordingly, it is capable of preventing or suppressing the hip-hold flaps HF from spreading outward in the widthwise direction when the rear area is folded inward (Fig. 1(a) and (b)).

The first-widthwise-direction folding line Lx1 may pass through any position in the lengthwise direction of the anti-slipping portions 9. However, when the first-widthwise-direction folding line Lx1 passes through the center of the lengthwise direction of the anti-slipping portions 9, flexibility of the hip-hold flaps HF in front of or behind of the first-widthwise-direction folding line Lx1 can be suitably reduced (the hip-hold flaps HF can be provided with a balanced stiffness in front of or behind of the first-widthwise-direction folding line Lx1). Therefore, when the first-widthwise-direction folding line Lx1 is folded, the hip-hold flap HF does not expand in the width direction.

In the embodiment illustrated in the drawings, the shape of the anti-slipping portions 9 is a rectangle having a longitudinal side in the lengthwise direction. However, the shape may be a square other than a rectangle or an elliptical shape. Further, the shape of the outer shape of the anti-slipping portions 9 may be along the outline of the hip-hold flaps HF. That is, the edge of the outer shape in the widthwise direction of the anti-slipping portions 9 may be curved while maintaining a curve with a certain distance from the edge of the hip-hold flaps HF.

Further, in the present embodiment, the first-widthwise-direction folding line Lx1 passes through the apexes 11P of the convex portions 11 of the hip-hold flaps HF. The first-widthwise-direction folding line Lx1 is a line extending substantially in the widthwise direction and may pass through the apexes 11P of one or both convex portions 11 of the two hip-hold flaps HF and HF. In the present specification, "passing through the apex of the convex portion" refers to passing through the apex and the vicinity of the convex portion. In an actual product, the folds are formed in a position that is preferably 8 mm or less and more preferably 5 mm or less in front of or behind from the apexes of the convex portions.

It is capable of folding at a position where the width of the folding is large (a position where the folded portion of the hip-hold flaps HF extends longer in the widthwise direction) as illustrated in FIG. 3B. Accordingly, when it is folded at the first-widthwise-direction folding line Lx1, the portion in front of or behind the first-widthwise-direction folding line Lx1 of the hip-hold flaps HF is difficult to move, thereby preventing the hip-hold flaps HF from spreading outward in the widthwise direction (FIG. 1).

In this manner, it is capable of preventing the hip-hold flaps HF from spreading outward in the widthwise direction because the first-widthwise-direction folding line Lx1 passes through the anti-slipping portions 9 and through the apexes 11P of the convex portions 11 of the hip-hold flaps HF. Therefore, it is capable of preventing the hip-hold flaps HF from unintentionally protruding from the wrapping sheet 20 or overlapping the sealing position of the wrapping sheet 20 when the edges of the wrapping sheet 20 are sealed after the absorbent article 1 is folded together with the wrapping sheet 20. Accordingly, the width of the wrapping sheet 20 is no longer required to be large in view of spreading outward in the widthwise direction of the hip-hold flaps HF, thus capable of providing a compact individually wrapped absorbent article.

In other words, the anti-slipping portions 9 are positioned so as to span a position of the apexes 11P of the convex portions 11 having the outline of the hip-hold flaps HF viewed in the lengthwise direction. Here, when the hip-hold flaps HF have a shape having a plurality of convex portions 11, the anti-slipping portions 9 are preferably arranged so as to span a position where the apexes of the widest convex portions are present. As a result, it is capable of folding at the widest position of the first-widthwise-direction folding line Lx1. Accordingly, the deformation and movement of the hip-hold flaps HF can be easily suppressed, and the hip-hold flaps spread outward in the widthwise direction can be effectively suppressed.

In a state illustrated in FIG. 3B, a peeling sheet 30 can be disposed on the anti-slipping portions 9. The peeling sheet 30 is a sheet for protecting the anti-slipping function (adhesiveness, high friction, or the like) of the anti-slipping portions 9. The peeling sheet 30 is a sheet that can open the individually wrapped absorbent article and peel off the absorbent article 1 immediately before use to expose the anti-slipping portions 9.

The peeling sheet 30 may be a sheet of paper; polyolefin-based films such as polypropylene, polyethylene, and the like; films such as polyester, polyvinyl alcohol, and the like; non-woven fabric; laminated non-woven fabric (including a non-woven polylaminated fabric); and the like, in which a mold-release treatment is applied to the surface abutting on the anti-slipping portions 9. The mold-release treatment can be formed by coating or spraying a mold-release treatment liquid such as silicone-based resin, fluorine-based resin, tetrafluoroethylene-based resin, and the like. Note that the peeling sheet is not particularly limited as long as the peeling sheet can be easily peeled off from the anti-slipping portions 9 without performing the mold-releasing treatment and does not cause a decrease in adhesion or high friction of the anti-slipping portions 9. Therefore, the peeling sheet may be a film itself or a non-woven fabric itself.

When the absorbent article 1 is folded at the first-widthwise-direction folding line Lx1, the peeling sheet 30 is also folded together with the hip-hold flaps HF and the anti-slipping portions 9 because the peeling sheet 30 is arranged to cover the anti-slipping portions 9. At this time, the hip-hold flaps HF and HF can be folded to the lengthwise direction while maintaining the plane shape because the peeling sheet 30 can hold the hip-hold flaps HF and HF in a plane shape along the portion in front of or behind of the first-widthwise-direction folding line Lx1. This further improves the aforementioned effect of suppressing the hip-hold flaps HF and the HF spreading outward in the widthwise direction (FIG. 1).

The peeling sheet 30 may have a rectangular shape as illustrated in FIG. 3B. However, the shape of the peeling sheet 30 is not particularly limited as long as the peeling sheet 30 can be disposed to cover the anti-slipping portions 9 in the folding process. Therefore, the shape of the peeling sheet is not limited to a rectangular shape but may be an elliptical shape. Alternatively, when anti-slipping portions are provided on the wing-like flaps W and W, the peeling sheet 30 may be a sheet that covers both the wing-like flaps W having anti-slipping portions and the hip-hold flaps HF having anti-slipping portions.

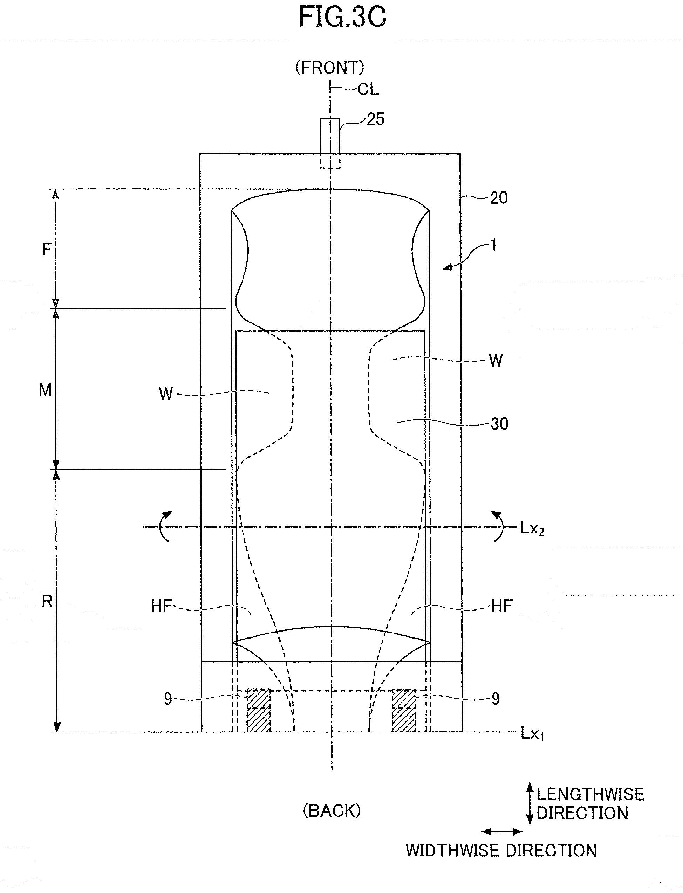

FIG. 3C illustrates a state after the absorbent article 1 is folded at the first-widthwise-direction folding line Lx1 illustrated in FIG. 3B. Then, the rear area R of the absorbent article 1 is folded with the top sheet 3 inward along a second-widthwise-direction folding line Lx2, which is positioned ahead of the first-widthwise-direction folding line Lx1. The second-widthwise-direction folding line Lx2 is preferably not included in the portion A corresponding to a body fluid excretion orifice, and more preferably not included in the middle area M.

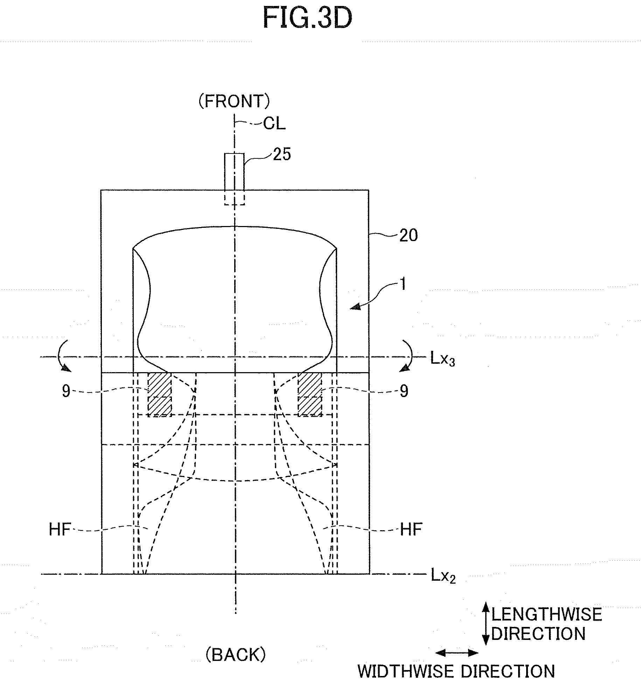

FIG. 3D illustrates a state of the absorbent article 1 after folding at the second-widthwise-direction folding line Lx2 illustrated in FIG. 3C. From the state of FIG. 3D, the area including the front end is folded with the top sheet 3 inward along a third-widthwise-direction folding line Lx3. A pouch tape 25 can then fasten the front end of the wrapping sheet 20.

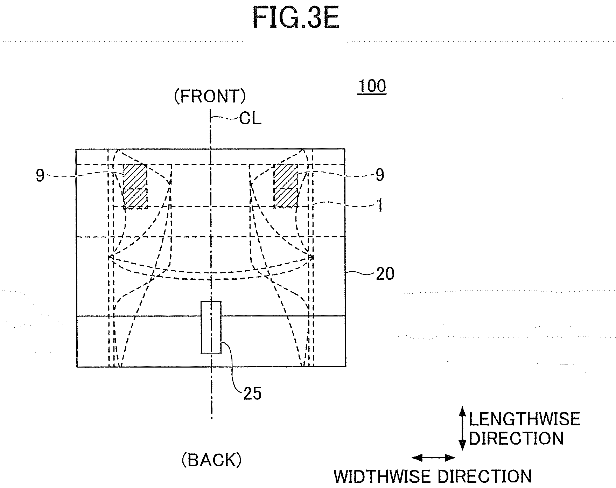

FIG. 3E illustrates an individually wrapped absorbent article 100 folded at the third-widthwise-direction folding line Lx3 illustrated in FIG. 3D. In this state, both edges of the wrapping sheet 20 can be sealed by heat sealing, ultrasonic sealing, or the like. According to the present embodiment, it is capable of preventing the hip-hold flaps HF and HF from unintentionally sealing together when sealing both edges of the above-described wrapping sheet 20 because the hip-hold flaps HF and HF are less likely to spread outward in the widthwise direction upon folding.

In the illustrated embodiment, the absorbent article 1 is folded toward the lengthwise direction inward along three widthwise directional lines that are substantially perpendicular to the lengthwise direction. However, folding lines extended in the widthwise direction may be two, four, or more. This makes it easier to carry and store the absorbent article 1 because the elongated absorbent article can be made into a compact shape with a shorter length.

### (Modified Examples of Individually Wrapped Absorbent Articles)

The main body 8 of the absorbent article 1 is provided with a flexible portion 50, and the first-widthwise-direction folding line Lx1 is formed as a line passing through the flexible portion 50 in the folding process described above. The flexible portion 50 is a portion that is more easily bent than the area around the flexible portion 50. The flexible portion 50 may be formed on at least one of the back sheet 2, the absorbent 4, or the top sheet 3. For example, the flexible portion 50 can be a thinned portion of the back sheet 2, the absorbent 4, and the top sheet 3. Hereinafter, the absorbent article 1 having the flexible portion 50 will be described.

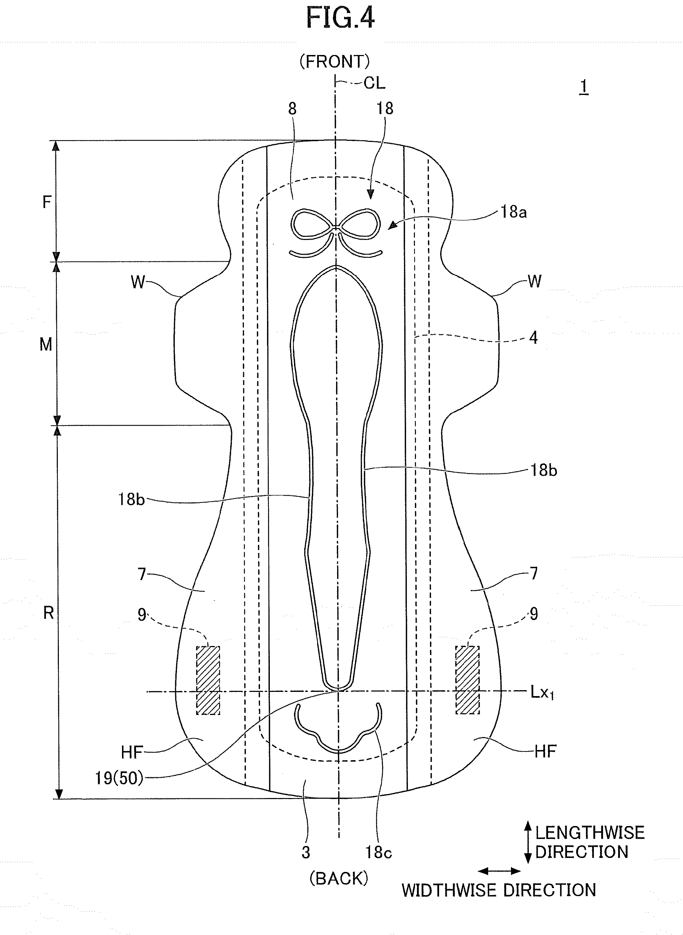

FIG. 4 illustrates, as an example, an absorbent article 1 having compressed grooves 18. The absorbent article illustrated in FIG. 4 has essentially the same configuration as the absorbent article illustrated in FIG. 2A, but differs in having the compressed grooves 18. As illustrated in FIG. 4, a plurality of compressed grooves 18 are formed across the front area F, the middle area M, and the rear area R. Also, the compressed grooves 18 are formed linearly symmetrical with the centerline CL in the lengthwise direction as a target axis. However, the compressed grooves 18 may not necessarily be formed in a linear symmetry. As illustrated in FIG. 4, the compressed grooves 18 may include a front compressed groove 18a, which is mainly provided in the front area F, a pair of central compressed grooves 18b, 18b extending in the lengthwise direction provided on both sides of the lengthwise centerline CL mainly from the middle area M to the rear area R, and a rear compressed groove 18c, which is provided near the rear end of the rear area R.

Central compressed grooves 18b and 18b, forming a pair, are connected to each other at the rear. In a link portion 19 of the pair of central compressed grooves 18b and 18b, both the top sheet 3 and the absorbent 4 are pressed, thereby the thickness of the link portion is reduced. Therefore, the main body 8 is easily bent at a position including the link portion 19. In this example, the first-widthwise-direction folding line Lx1 can be folded through the link portion 19 of the pair of central compressed grooves 18b and 18b upon folding (FIGS. 3A to 3E). As a result, folding at the first-widthwise-direction folding line Lx1 (FIG. 3B) becomes easier, and it is capable of stably maintaining the folded state even after folding.

Thus, even if vibration or force is applied to the absorbent article 1 from the outside and the vibration or force is transmitted to the hip-hold flaps HF and HF, the hip-hold flaps HF and HF do not easily deform and can be maintained in a folded state after the absorbent article 1 is folded at the first-widthwise-direction folding line Lx1 in the individually wrapping process (folding process) of the absorbent article 1. Therefore, the effect of preventing the hip-hold flaps HF and HF from spreading outward in the widthwise direction can be further enhanced.

The link portion 19 of the pair of central compressed grooves 18b and 18b may be a compressed groove extending in the widthwise direction. In this case, the main body 8 can be more easily bent and more stably retained in a bent state by aligning the first-widthwise-direction folding line Lx1 along the compressed groove.

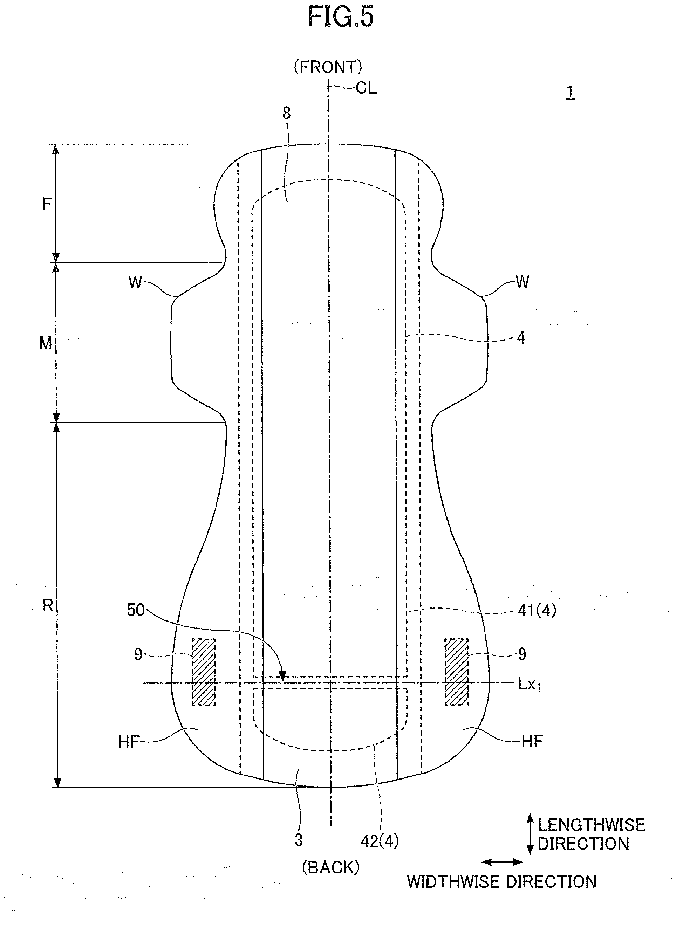

Another modified example of absorbent article 1 is illustrated in FIG. 5. The example illustrated in FIG. 5 has the same configuration as that of the absorbent article 1 illustrated in FIG. 2A, except that a flexible portion 50 is provided in the absorbent 4. As illustrated in FIG. 5, the main body 8 has the flexible portion 50 in the rear area R. In the present example, the flexible portion 50 is a portion in which the absorbent 4 is not provided. That is, the absorbent 4 is divided into an absorbent portion 41 and an absorbent portion 42 disposed behind the absorbent portion 41. The flexible portion 50 is formed between the absorbent portions.

In the illustrated example, the flexible portion 50 is a portion in which the absorbent 4 is not provided for the entire width. However, a configuration of the flexible portion 50 may alternately have a portion in which the absorbent 4 is present over the widthwise direction and have a portion in which the absorbent 4 is not present over the widthwise direction.

In this example, it is capable of folding at the first-widthwise-direction folding line Lx1 through the flexible portion 50. That is, it is capable of folding between the absorbent portions 41 and 42 upon folding (FIGS. 3A to 3E). As a result, folding at the first-widthwise-direction folding line Lx1 (FIG. 3B) becomes easier, and it is capable of stably maintaining the folded state even after folding.

In the illustrated example, the flexible portion 50 is a portion without the absorbent, but the thickness of the absorbent may be smaller than the front and rear portions thereof.

Hereinafter, preferred embodiments of the present invention will be described.

### DESCRIPTION OF THE REFERENCE NUMERALS

1: Absorbent article
2: Back sheet
3: Top sheet
4: Absorbent
7: Side non-woven fabric
8: Main body of absorbent article
9: Anti-slipping portion
11: Convex portion
11P: Apex of convex portion
18, 18a, 18b, and 18c: Compressed grooves
19: Link portion of central compressed groove
20: Wrapping sheet
25: Pouch tape
30: Peeling sheet
41, 42: Absorbent portions
50: Flexible portion
100: Individually wrapped absorbent article
A: Portion corresponding to a body fluid excretion orifice
CL: Centerline in lengthwise direction
F: Front area
HF: Hip-hold flap
M: Middle area
R: Rear area
W: Wing-like flap

## Claims

1. An individually wrapped absorbent article (100) comprising:
an absorbent article (1) being a sanitary napkin, the absorbent article (1) having a main body (8), the main body (8) including a liquid-impermeable back sheet (2), an absorbent (4), and a liquid-permeable top sheet (3) that are laminated in aforementioned order, the main body (8) is the absorbent article (1) having a predetermined length in a lengthwise direction and a predetermined width in a direction perpendicular to the lengthwise direction; and
a wrapping sheet (20) that wraps the absorbent article (1) individually,
wherein the absorbent article (1) includes a middle area (M) that includes a portion (A) corresponding to a body fluid excretion orifice of a wearer upon wearing the absorbent article (1), and a rear area (R) that is adjacently behind the middle area (M) and extends to a rear end of the absorbent article (1),
wherein the absorbent article (1) includes hip-hold flaps (HF) projecting laterally from the main body (8) in the rear area (R), and an outline of the hip-hold flaps (HF) has convex portions with a radius of curvature of 5 to 500 mm,
wherein anti-slipping portions (9) are disposed on a back surface of the hip-hold flaps (HF),
wherein the hip-hold flaps (HF) are folded with a top sheet side inward along lengthwise direction folding lines extending substantially in the lengthwise direction, and the rear area (R) is then folded with the top sheet side inward along a widthwise direction folding line extending substantially in the widthwise direction through the anti-slipping portions (9) and apexes (11P) of the convex portions,
wherein a flexible portion (50) is formed in the main body (8), and the widthwise direction folding line passing through the flexible portion (50), and
wherein the flexible portion (50) includes a portion having a reduced thickness of the absorbent (4).

2. The individually wrapped absorbent article (100) according to claim 1, wherein the apexes (11P) are positioned rearward of the center in the rear area (R) in the lengthwise direction.

3. The individually wrapped absorbent article (100) according to claim 1, further comprising a peeling sheet (30) positioned so as to overlap exposed anti-slipping portions (9), in which the exposed anti-slipping portions (9) are formed by folding the hip-hold flaps (HF) with the top sheet side inward along the lengthwise direction folding lines,
wherein the peeling sheet (30) is folded together with the hip-hold flaps (HF) at the widthwise direction folding line.

## Patentansprüche

1. Ein einzeln eingepackter absorbierender Artikel (100), umfassend:
einen absorbierenden Artikel (1), bei dem es sich um eine Damenbinde handelt, wobei der absorbierende Artikel (1) einen Hauptkörper (8) aufweist, wobei der Hauptkörper (8) eine flüssigkeitsundurchlässige Rückschicht (2), ein Absorptionsmittel (4) und eine flüssigkeitsdurchlässige Deckschicht (3) umfasst, die in der vorgenannten Reihenfolge laminiert sind, wobei der Hauptkörper (8) der absorbierende Artikel (1) ist, der eine vorbestimmte Länge in einer Längsrichtung und eine vorbestimmte Breite in einer Richtung senkrecht zur Längsrichtung aufweist; und
eine Umhüllungslage (20), die den absorbierenden Artikel (1) individuell einhüllt,
wobei der absorbierende Artikel (1) einen mittleren Bereich (M) aufweist, der einen Abschnitt (A) aufweist, der einer Körperflüssigkeitsausscheidungsöffnung eines Trägers beim Tragen des absorbierenden Artikels (1) entspricht, und einen hinteren Bereich (R), der sich angrenzend hinter dem mittleren Bereich (M) befindet und sich zu einem hinteren Ende des absorbierenden Artikels (1) erstreckt,
wobei der absorbierende Artikel (1) Hüfthaltelaschen (HF) umfasst, die seitlich von dem Hauptkörper (8) in dem hinteren Bereich (R) vorstehen, und ein Umriss der Hüftaltelaschen (HF) konvexe Abschnitte mit einem Krümmungsradius von 5 bis 500 mm aufweist,
wobei Anti-Rutsch-Abschnitte (9) auf einer hinteren Fläche der Hüfthaltelaschen (HF) angeordnet sind,
wobei die Hüfthaltelaschen (HF) mit einer oberen Blattseite nach innen entlang von Falzlinien in Längsrichtung gefaltet werden, die sich im Wesentlichen in der Längsrichtung erstrecken, und der hintere Bereich (R) dann mit der oberen Blattseite nach innen entlang einer Falzlinie in Breitenrichtung gefaltet wird, die sich im Wesentlichen in der Breitenrichtung durch die Anti-Rutsch-Abschnitte (9) und die Scheitelpunkte (11P) der konvexen Abschnitte erstreckt,
wobei ein flexibler Abschnitt (50) in dem Hauptkörper (8) ausgebildet ist, und die Faltlinie in Breitenrichtung durch den flexiblen Abschnitt (50) verläuft, und
wobei der flexible Abschnitt (50) einen Abschnitt mit einer reduzierten Dicke des Absorptionsmittels (4) aufweist.

2. Der einzeln eingepackte absorbierende Artikel (100) nach Anspruch 1, wobei die Scheitelpunkte (11P) in der Längsrichtung hinter der Mitte im hinteren Bereich (R) angeordnet sind.

3. Der einzeln eingepackte absorbierende Artikel (100) nach Anspruch 1, der ferner eine Abziehlage (30) umfasst, die so positioniert ist, dass sie freiliegende Antirutschabschnitte (9) überlappt, wobei die freiliegenden Antirutschabschnitte (9) durch Falten der Hüfthaltelaschen (HF) mit der oberen Lagenseite nach innen entlang der Faltlinien in Längsrichtung gebildet werden,
wobei die Abziehlage (30) zusammen mit den Hüfthaltelaschen (HF) an der Falzlinie in Breitenrichtung gefaltet wird.

## Revendications

1. Article absorbant emballé individuellement (100) comprenant :
un article absorbant (1) qui est une serviette hygiénique, l'article absorbant (1) présentant un corps principal (8), le corps principal (8) incluant une feuille verso (2) imperméable aux liquides, un absorbant (4), et une feuille de dessus (3) perméable aux liquides qui sont stratifiés dans l'ordre susmentionné, le corps principal (8) est l'article absorbant (1) ayant une longueur prédéterminée dans une direction de longueur et une largeur prédéterminée dans une direction perpendiculaire à la direction de longueur ; et
une feuille d'emballage (20) qui emballe l'article absorbant (1) individuellement,
sachant que l'article absorbant (1) inclut une zone médiane (M) qui inclut une partie (A) correspondant à un orifice d'excrétion de fluide corporel d'une porteuse lors du port de l'article absorbant (1), et une zone arrière (R) qui est située de manière adjacente derrière la zone médiane (M) et s'étend à une extrémité arrière de l'article absorbant (1),
sachant que l'article absorbant (1) inclut des volets de maintien de hanche (8) dépassant latéralement du corps principal (8) dans la zone arrière (R), et un contour des volets de maintien de hanche (HF) présente des parties convexes ayant un rayon de courbure de 5 à 500 mm,
sachant que des parties antiglissement (9) sont disposées sur une surface verso des volets de maintien de hanche (HF),
sachant que les volets de maintien de hanche (HF) sont pliés avec un côté de feuille de dessus vers l'intérieur le long de lignes de pliage de direction de longueur s'étendant sensiblement dans la direction de longueur, et la zone arrière (R) est ensuite pliée avec le côté de feuille de dessus vers l'intérieur le long d'une ligne de pliage de direction de largeur s'étendant sensiblement dans la direction de largeur via les parties antiglissement (9) et des sommets (11P) des parties convexes,
sachant qu'une partie flexible (50) est formée dans le corps principal (8), et la ligne de pliage de direction de largeur passant par la partie flexible (50), et
sachant que la partie flexible (50) inclut une partie ayant une épaisseur réduite de l'absorbant (4).

2. L'article absorbant emballé individuellement (100) selon la revendication 1, sachant que les sommets (11P) sont positionnés vers l'arrière du centre dans la zone arrière (R) dans la direction de longueur.

3. L'article absorbant emballé individuellement (100) selon la revendication 1, comprenant en outre une feuille détachable (30) positionnée de manière à chevaucher des parties antiglissement exposées (9), les parties antiglissement exposées (9) étant formées par pliage des volets de maintien de hanche (HF) avec le côté de feuille de dessus vers l'intérieur le long des lignes de pliage de direction de longueur,
sachant que la feuille détachable (30) est pliée conjointement avec les volets de maintien de hanche (HF) au niveau de la ligne de pliage de direction de largeur.
